# FASCICULE DE BREVET EUROPEEN

(11) **EP 0 361 995 B1**
(45) Date de publication et mention de la délivrance du brevet: **12.05.1993**
(21) Numéro de dépôt: 89402297.9
(22) Date de dépôt: 17.08.1989
(51) Int. Cl.: C07D 279/20, C07D 513/04, C07D 293/10, C07C 319/02, C07C 321/26, C07C 391/02

(54) **Procédé de préparation de diarylsulfures ou de diarylsélénures**
Verfahren zur Herstellung von Diarylschwefel- oder Diarylselen-Verbindungen
Process for the preparation of diarylsulfide or diarylselene compounds

(30) Priorité: 18.08.1988 FR 8810982
(43) Date de publication de la demande: 04.04.1990
(73) Titulaire: RHONE-POULENC SANTE, 92160 Antony (FR)
(72) Inventeur: Duchesne, Jean-Pierre, F-69007 Lyon (FR)
(74) Mandataire: Pilard, Jacques

(56) Documents cités:
- DE-C- 964 050
- FR-A- 1 029 987
- FR-A- 1 058 936
- US-A- 2 433 658
- CHEMICAL ABSTRACTS, vol. 98, no. 23, 6 juin 1983, page 645, résumé no. 198127m, Columbus, Ohio, US; D. BLACKBURN et al.: "Synthesis of sulfur-35 labeled phenothiazines"
- CHEMICAL ABSTRACTS, vol. 103, no. 23, 9 décembre 1985, page 637, résumé no. 195669a, Columbus, Ohio, US; V.V. FILINOVA et al.: "Kinetics of the reaction of diphenylamine with sulfur"
- CHEMICAL ABSTRACTS, vol. 107, no. 22, 30 novembre 1987, page 5, résumé no. 199004k, Columbus, Ohio, US; V.A. SERGEEV et al.: "Synthesis, structure, and electrophysical properties of poly(phenyleneaminophenylene sulfide)"
- CHEMICAL ABSTRACTS, vol. 73, 6th July 1970, page 335, résumé 3895p, Columbus, Ohio, US; B.D. PODOLESOV et al.: "Preparation of 10-aroylphenoselenazines"

## Description

La présente invention concerne la préparation d'hétérocycles condensés soufrés ou séléniés éventuellement substitués de formule générale : 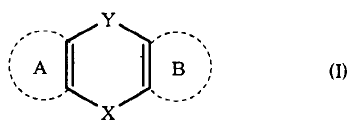
dans laquelle les cycles A et B, identiques ou différents représentent un cycle phényle ou un cycle hétérocyclique aromatique contenant 1 ou 2 atomes d'azote tel pyridyle ou pyrimidyle, X représente un atome de soufre ou de sélénium et Y représente un groupement -NH- par action du soufre et du dioxyde de soufre (S0₂) ou du sélénium et du dioxyde de sélénium (SeO₂) sur un produit de formule générale : 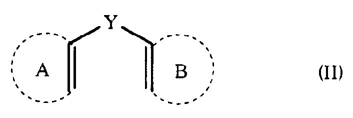
dans laquelle les cycles A et B et le symbole Y sont définis comme précédemment.

Plus particulièrement encore, la présente invention concerne la préparation de phénothiazines ou de phénosélénazines de formule générale : 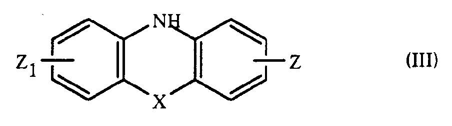
dans laquelle X est défini comme précédemment, et Z et Z₁, identiques ou différents, représentent un ou plusieurs atomes ou radicaux choisis parmi les atomes d'hydrogène ou d'halogène et les radicaux alkyle, alkoxy, hydroxy, amino, alkylamino, dialkylamino, acyle, acylamino, les radicaux alkyles et les portions alkyles des autres radicaux contenant 1 à 4 atomes de carbone et pouvant être substitué par un radical phényle, par action du soufre et du dioxyde de soufre ou du sélénium et du dioxyde de sélénium sur un produit de formule générale: 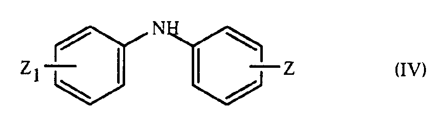
dans laquelle Z et Z₁ sont défnis comme précédemment.

Il est connu que le soufre ou le sélénium réagissent avec les composés aromatiques selon le schéma suivant:

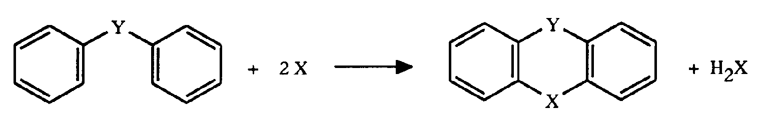

La réaction est catalysée généralement par des halogènes (brome, iode), les halogénures d'aluminium (chlorure, bromure, iodure), les halogénures ferriques (chlorure, bromure), le chlorure de zinc ou le chlorure d'antimoine comme cela a été décrit, par exemple, dans les brevets français FR 1 029 987 ou FR 1 058 936 ou dans les brevets américains US 2 415 363 ou US 2 433 658.

Selon les procédés connus les phénothiazines peuvent être obtenues en faisant réagir deux moles de soufre par mole de diphénylamine en présence d'un catalyseur dont la quantité est généralement inférieure à 3 % en poids. La réaction s'effectue à une température supérieure à 120°C. La cinétique de la réaction s'accroît avec l'élévation de la température et il peut être avantageux d'opérer à une température comprise entre 180 et 200°C, une température supérieure à 200°C conduisant à la formation de goudrons et de produits secondaires indésirables. Par ailleurs, la mise en oeuvre des procédés connus conduit à la formation d'une quantité stoechiométrique d'hydrogène sulfuré (H₂S).

Généralement, lors de la mise en oeuvre des procédés connus, la vitesse de réaction diminue considérablement lorsque le taux de transformation de la diphénylamine atteint 90 %. Pour obtenir une réaction complète, il est alors nécessaire d'élever la température et d'éliminer l'hydrogène sulfuré au moyen, par exemple, d'un gaz inerte tel que l'azote, l'argon ou le gaz carbonique. Parfois le ralentissement de la vitesse de réaction peut être dû à une perte de catalyseur qui est entraîné dans les effluents. Ainsi, lorsque l'on utilise de l'iode, qui est le catalyseur généralement utilisé dans ce type de réaction, il se produit une perte sous forme d'acide iodhydrique. Il est donc nécessaire d'augmenter la quantité de catalyseur ou même d'ajouter le catalyseur en continu.

Cependant, le principal inconvénient des procédés connus réside dans la production d'hydrogène sulfuré. En dehors du fait que la formation de l'hydrogène sulfuré consomme la moitié du soufre mis en oeuvre, il est nécessaire de l'éliminer ce qui entraîne, pour des raisons d'hygiène industrielle et de protection de l'environnement, l'utilisation d'appareils industriels coûteux.

Il a été proposé de remplacer le soufre par des composés soufrés tels que le chlorure de soufre, le dichlorure de soufre, le thiosulfate de sodium, le sulfure d'antimoine ou d'arsenic, les polysulfures alcalins ou le chlorure de thionyle qui conduisent à une formation moins importante d'hydrogène sulfuré. Cependant, les rendements sont alors généralement médiocres et les produits obtenus nécessitent des purifications longues et difficiles.

Le procédé selon la présente invention permet d'obtenir des sulfures ou des sélénures aromatiques sans formation d'hydrogène sulfuré ou d'hydrogène sélénié avec de très bons rendements et avec des vitesses de réaction élevées.

Le procédé selon l'invention consiste à traiter dans un milieu fermé un composé aromatique de formule (II) ou (IV) dans laquelle les différents symboles sont définis comme précédemment, par un mélange de soufre et de dioxyde de soufre ou de sélénium et de dioxyde de sélénium, le dioxyde de soufre ou le dioxyde de sélénium étant utilisés en quantité suffisante pour transformer l'hydrogène sulfuré ou l'hydrogène sélénié en soufre ou en sélénium.

Le soufre ou le sélénium ainsi régénérés participent à nouveau à la réaction jusqu'à sa disparition complète. La réaction peut être pratiquement quantitative sans que des traces d'hydrogène sulfuré ou d'hydrogène sélénié puissent être décelées. Il en résulte que le procédé selon l'invention peut être schématisé par les équations suivantes :

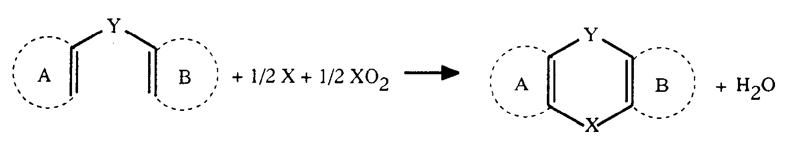

Il en résulte que, par la mise en oeuvre du procédé selon l'invention, la totalité du soufre engagé sous forme S₈ ou du sélénium ou de dioxyde de soufre ou de dioxyde de sélénium est consommée et que le sous-produit de la réaction est l'eau dont l'élimination ne soulève pas de problèmes particuliers.

Pour la mise en oeuvre du procédé selon l'invention, il est nécessaire d'utiliser une quantité stoechiométrique de soufre ou de sélénium par rapport au dérivé aromatique mis en oeuvre. Par exemple, dans le cas de la synthèse de phénothiazines ou de phénosélénazines de formule générale (V), on utilise 0,5 mole de soufre ou de sélénium par mole de diphénylamine de formule générale (VI) mise en oeuvre. En utilisant une quantité inférieure, la réaction est incomplète et en utilisant une quantité supérieure, il se forme des produits secondaires indésirables provenant de la réaction du soufre ou du sélénium sur la phénothiazine ou de phénosélénazine obtenue.

Pour la mise en oeuvre du procédé selon l'invention, il est nécessaire d'utiliser une quantité au moins stoechiométrique de dioxyde de soufre ou de dioxyde de sélénium par rapport au dérivé aromatique mis en oeuvre. Par exemple, dans le cas de la synthèse de phénothiazines ou de phénosélénazines de formule générale (III), on utilise au moins 0,5 mole de dioxyde de soufre ou de dioxyde de sélénium par mole de diphénylamine de formule générale (IV) mise en oeuvre. Généralement, un excès de 5 à 20 % en mole de dioxyde de soufre ou de dioxyde de sélénium permet d'augmenter la vitesse de réaction sans nuire à la sélectivité.

Le dioxyde de soufre peut être utilisé soit sous forme liquide, soit sous forme gazeuse en opérant sous pression, soit en solution dans un solvant organique convenable. Cependant le procédé ne peut pas être mis en oeuvre en opérant dans le dioxyde de soufre liquide seul.

Le dioxyde de sélénium est généralement utilisé sous forme solide ou en solution dans un solvant organique convenable.

Comme catalyseurs peuvent être utilisés les halogénures d'aluminium (chlorure, bromure,iodure) les halogènures de gallium (chlorure, bromure), l'iodure de lithium, les iodures d'ammonium quaternaires, les iodures de sodium et de potassium en présence d'un acide fort tel que l'acide phosphorique, les iodures des métaux de transition (fer, chrome, cobalt), l'iodure de cuivre, et plus généralement les composés permettant de libérer un iodure ou de l'iode, dans les conditions de la réaction, le brome, l'acide bromhydrique, l'acide iodhydrique, le trifluorure de bore et de préférence l'iode. Généralement, on utilise une quantité de catalyseur représentant de 0,04 à 50 % en mole par rapport au produit de départ utilisé, la quantité de catalyseur utilisé pouvant être fonction de sa sensibilité à l'eau libérée au cours de la réaction.

Généralement, en utilisant l'iode comme catalyseur, la vitesse de la réaction est liée directement à la quantité d'iode utilisée et, dans le cas de la préparation de composés cycliques substitués pouvant exister sous plusieurs formes isomériques, la sélectivité peut diminuer en fonction de l'augmentation de la quantité d'iode utilisée. Par ailleurs, la nature des substituants peut influer sur le déroulement de la réaction. Ainsi, les substituants électro-donneurs ont tendance à favoriser la réaction et, de ce fait, il peut être possible de mettre en oeuvre le procédé avec des quantités plus faibles de catalyseur.

Pour la mise en oeuvre du procédé selon l'invention, la température est supérieure à 80°C et est généralement comprise entre 130 et 280°C. Dans le cas de la préparation des phénothiazines de formule générale (V), il est particulièrement important d'opérer à une température inférieure à 200°C afin d'éviter la formation de produits secondaires indésirables. Généralement, la température optimale de la réaction sera fonction de la nature du composé aromatique mis en oeuvre, de la nature et de la quantité de catalyseur utilisé et de la mise en oeuvre du procédé en présence ou non de solvant.

Dans le cas particulier de la cyclisation de diphénylamines de formule générale (IV) dont un des noyaux phényles est substitué en position -3, la mise en oeuvre du procédé conduit généralement à la phénothiazine substituée en position -2 (nomenclature de l'I.U.P.A.C.) losqu'on opère à basse température et à la phénothiazine substituée en position -4 lorsqu'on opère à température élevée. Selon la température de réaction il est possible d'obtenir un mélange des isomères en positions -2 et -4.

Généralement, le procédé est mis en oeuvre dans la masse des réactifs maintenus à l'état liquide à la température de réaction. Cependant, il peut être avantageux d'opérer dans un solvant convenable qui possède la propriété de dissoudre les composés aromatiques de départ, les réactifs et les produits de la réaction afin d'obtenir un milieu homogène. Généralement les solvants sont choisis parmi les solvants qui sont inertes chimiquement dans les conditions de mise en oeuvre du procédé. Les solvants qui conviennent particulièrement bien sont choisis parmi les hydrocarbures aliphatiques ou alicycliques saturés contenant 10 à 16 atomes de carbone tels que la décaline ou le bicyclohexyle éventuellement substitués par un ou plusieurs radicaux alkyles contenant 1 à 4 atomes de carbone, ou, de préférence, parmi les solvants aprotiques polaires non basiques tels que les dialkylsulfones contenant moins de 10 atomes de carbone ou le sulfolane (tétrahydrothiophène dioxyde-1,1).

Généralement, le procédé est mis en oeuvre dans un réacteur fermé sous pression autogène qui, selon les conditions de réaction, est inférieure à 5 bars en début de réaction.

En fonction de la nature et de la quantité des produits mis en oeuvre, de la température et de la nature du solvant, la durée de réaction peut être comprise entre 30 minutes et 5 heures.

Les produits de la réaction sont isolés par application des méthodes habituelles telles que précipitation et recristallisation dans un solvant convenable.

Les produits obtenus par la mise en oeuvre du procédé selon l'invention, et en particulier les phénothiazines et les phénosélénazines, sont particulièrement utiles pour préparer des produits thérapeutiquement actifs.

Les exemples suivants, donnés à titre non limitatif, montrent comment l'invention peut être mise en pratique.

### EXEMPLE 1

Dans un tube de 25 cm3, on introduit sous atmosphère d'azote, en solution dans 4 cm3 de sulfolane, 1,00 g de méthoxy-3 diphénylamine (5 mmoles), 0,08 g de soufre (2,5 mmoles), 0,2 g de dioxyde de soufre (3,12 mmoles) et 0,00254 g d'iode (0,001 mmole). Le tube est scellé puis chauffé pendant 2 heures à 165°C.

Le mélange réactionnel est dilué par 15 cm3 d'eau. Le produit qui précipite est séparé par filtration. On obtient ainsi un produit contenant 0,958 g (83 %) de méthoxy-2 phénothiazine.

Dans les eaux-mères, on dose :
- 0,003 g de méthoxy-3 diphénylamine
- 0,050 g de méthoxy-4 phénothiazine.

Le taux de transformation de la méthoxy-3 diphénylamine est de 91 %.

Le rendement chimique en méthoxy-2 phénothiazine est de 91 %.

### EXEMPLE 2

Dans un tube de 25 cm3, on introduit sous atmosphère d'azote, en solution dans 3 cm3 de solfolane, 1 g de diphénylamine (5,92 mmoles), 0,095 g de soufre (2,97 mmoles), 0,230 g de dioxyde de soufre (3,60 mmoles) et 0,004 g d'iode. Le tube est scellé puis chauffé pendant 5 heures à 180°C sous pression autogène.

Après refroidissement, le mélange est repris par du chlorure de méthylène. Par chromatographie liquide à haute performance (CLHP), on dose :
- 0,070 g de diphénylamine
- 1,09 g de phénothiazine.

Le taux de transformation de la diphénylamine est de 93 %.

Le rendement en phénothiazine est de 99 % par rapport à la diphénylamine transformée.

### EXEMPLE 3

Dans un tube de 25 cm3, on introduit sous atmosphère d'azote, en solution dans 1 cm3 de sulfolane, 1,00 g de chloro-3 diphénylamine (4,99 mmoles), 0,080 g de soufre (2,50 mmoles), 0,190 g de dioxyde de soufre (2,97 mmoles) et 0,0125 g d'iode (0,049 mmoles). Le tube est scellé puis chauffé pendant 4 heures à 160°C sous pression autogène. Après refroidissement, le mélange est repris par du chlorure de méthylène.

Par chromatographie liquide à haute performance, on dose :
- 0,200 g de chloro-3 diphénylamine
- 0,579 g de chloro-2 phénothiazine
- 0,248 g de chloro-4 phénothiazine.

Le taux de transformation de la chloro-3 diphénylamine est de 80 %.

Le rendement en chloro-2 phénothiazine est de 63 % par rapport à la chloro-3 diphénylamine transformée.

### EXEMPLE 4

Dans un tube de 25 cm3, on introduit sous atmosphère d'azote, en solution dans 4 cm3 de toluène, 0,510 g de N-phénylamino-3 pyridine (3,0 mmoles), 0,048 g de soufre (1,50 mmoles), 0,145 g de dioxyde de soufre et 0,0076 g d'iode (0,03 mmoles). Le tube est scellé puis chauffé pendant 2 heures à 260°C sous pression autogène. Après refroidissement l'analyse du mélange réactionnel par résonance magnétique nucléaire à 200 MHz montre que :
- le taux de transformation de la N-phénylamino-3 pyridine est voisin de 50 %,
- le rendement en aza-4 phénothiazine est voisin de 20 %,
- le rendement en aza-2 phénothiazine est voisin de 20 %.

### EXEMPLE 5

Dans un tube de 25 cm3, on introduit, sous atmosphère inerte, 0,506 g (3 mmoles) de diphénylamine, 0,200 g (1,80 mmole) de dioxyde de sélénium (SeO₂) et 0,119 g (1,5 mmole) de sélénium et 25 mg (0,1 mmole) d'iode en solution dans 4 cm3 de sulfolane.

Le tube est scellé puis chauffé pendant 4 heures à 200°C.

Le mélange réactionnel est repris par un mélange éthanol-chlorure de méthylène. Le dosage par chromatographie liquide à haute performance (CLHP) montre que le taux de transformation est voisin de 40 % et que le rendement en phénosélénazine est de 38 %.

## Revendications

1. Procédé pour la préparation d'hétérocycles condensés soufrés ou séléniés éventuellement substitués de formule générale : 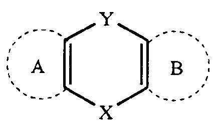 dans laquelle les cycles A et B, identiques ou différents représentent un cycle phényle ou un cycle hétérocyclique aromatique contenant 1 ou 2 atomes d'azote et X représente un atome de soufre ou de sélénium et Y représente un groupement -NH-caractérisé en ce que, sur un produit de formule générale : 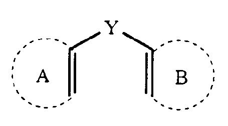 dans laquelle les cycles A et B et le symbole X sont définis comme précédemment, on fait réagir 1/2 équivalent de soufre ou de sélénium par mole de phénothiazine ou de phénosélénazine formé et au moins 1/2 équivalent de dioxyde de soufre ou de dioxyde de sélénium par mole de phénothiazine ou de phénosélénazine formée en opérant à une température supérieure à 80°C, et isole le produit obtenu.

2. Procédé selon la revendication 1 caractérisé en ce que l'on opère en présence d'un catalyseur choisi parmi les halogènures d'aluminium ou de gallium, les iodures de lithium, de sodium, de potassium ou d'ammonium quaternaires, les iodures de fer, de chrome, de cobalt ou de cuivre et les composés permettant de libérer un iodure ou de l'iode dans les conditions de la réaction, les acides bromhydrique et iodhydrique, le brome et l'iode.

3. Procédé selon la revendication 2 caractérisé en ce que le catalyseur est l'iode.

4. Procédé selon l'une des revendications 1, 2 ou 3 caractérisé en ce que l'on opère dans un solvant organique choisi parmi les hydrocarbures aliphatiques ou cycloaliphatiques saturés contenant 10 à 16 atomes de carbone et les solvants aprotiques polaires non basiques choisis parmi les dialkylsulfones contenant moins de 10 atomes de carbone ou le sulfolane.

5. Procédé selon l'une des revendications 1, 2, 3 ou 4 caractérisé en ce que l'on opère à une température comprise entre 130 et 280°C.

6. Procédé selon l'une des revendications 1, 2, 3, 4 ou 5 caractérisé en ce que l'on opère sous pression autogène.

7. Procédé selon l'une des revendications 1, 2, 3, 4, 5 ou 6 pour la préparation de phénothiazines ou de phénosélénazines de formule générale : 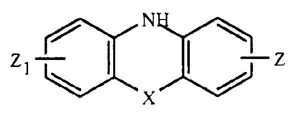 dans laquelle X représente un atome de soufre ou de sélénium et Z et Z₁, identiques ou différents représentent un ou plusieurs atomes ou radicaux choisis parmi les atomes d'hydrogène ou d'halogène et les radicaux alkyle, alkoxy, hydroxy, amino, alkylamino, dialkylamino, acyle, acylamino, les radicaux alkyles et les portions alkyles des autres radicaux contenant 1 à 4 atomes de carbone et pouvant être substitué par un radical phényle, par action du soufre et du dioxyde de soufre ou du sélénium et du dioxyde de sélénium sur un produit de formule générale : 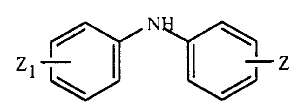 dans laquelle Z et Z₁ sont défnis comme précédemment.

## Patentansprüche

1. Verfahren zur Herstellung von gegebenenfalls substituierten schwefel- oder selenhaltigen kondensierten Heterocyclen der allgemeinen Formel 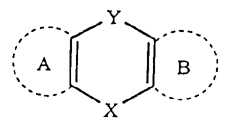 worin die Ringe A und B, die identisch oder voneinander verschieden sind, einen Phenylring oder einen aromatischen heterocyclischen Ring mit 1 oder 2 Stickstoffatomen bedeuten, und X für ein Schwefel- oder Selenatom steht, und Y eine Gruppe -NH- wiedergibt, dadurch gekennzeichnet, daß man mit einem Produkt der allgemeinen Formel 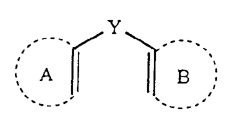 worin die Ringe A und B und das Symbol X wie vorstehend definiert sind, 1/2 Äquivalent Schwefel oder Selen je Mol gebildetes Phenothiazin oder Phenoselenazin und zumindest 1/2 Äquivalent Schwefeldioxid oder Selendioxid je Mol gebildetes Phenothiazin oder Phenoselenazin umsetzt, wobei man bei einer Temperatur oberhalb 80°C arbeitet, und das erhaltene Produkt isoliert.

2. Verfahren gemäß Anspruch 1, dadurch gekennzeichnet, daß man in Gegenwart eines Katalysators, ausgewählt unter den Aluminium- oder Galliumhalogeniden, den Lithium-, Natrium-, Kalium- oder quaternären Ammoniumjodiden, den Eisen-, Chrom-, Kobalt- oder Kupferjodiden und den Verbindungen, die es erlauben, unter den Reaktionsbedingungen Jodid oder Jod freizusetzen, den Bromwasserstoff- und Jodwasserstoffsäuren, dem Brom und Jod,arbeitet.

3. Verfahren gemäß Anspruch 2, dadurch gekennzeichnet, daß der Katalysator Jod ist.

4. Verfahren gemäß einem der Ansprüche 1, 2 oder 3, dadurch gekennzeichnet, daß man in einem organischen Lösungsmittel, ausgewählt unter den gesättigten aliphatischen oder cycloaliphatischen Kohlenwasserstoffen mit 10 bis 16 Kohlenstoffatomen und den nicht-basischen polaren aprotischen Lösungsmitteln, ausgewählt unter den Dialkylsulfonen, die weniger als 10 Kohlenstoffatome enthalten, oder dem Sulfolan arbeitet.

5. Verfahren gemäß einem der Ansprüche 1, 2, 3 oder 4, dadurch gekennzeichnet, daß man bei einer Temperatur zwischen 130 und 280°C arbeitet.

6. Verfahren gemäß einem der Ansprüche 1, 2, 3, 4 oder 5, dadurch gekennzeichnet, daß man unter autogenem Druck arbeitet.

7. Verfahren gemäß einem der Ansprüche 1, 2, 3, 4, 5 oder 6 zur Herstellung von Phenothiazinen oder Phenoselenazinen der allgemeinen Formel 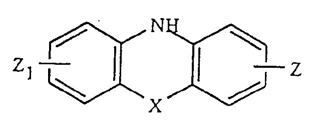 worin X für ein Schwefel oder Selenatom steht, und Z und Z₁, die gleich oder voneinander verschieden sind, einen oder mehrere Atome oder Reste wiedergeben, ausgewählt unter den Wasserstoff- oder Halogenatomen, und den Alkyl-, Alkoxy, Hydroxy-, Amino-, Alkylamino-, Dialkylamino-, Acyl-, Acylaminoresten, wobei die Alkylreste und die Alkylteile der anderen Reste 1 bis 4 Kohlenstoffatome enthalten und durch einen Phenylrest substituiert sein können, durch Umsetzung von Schwefel oder Schwefeldioxid oder von Selen oder Selendioxid mit einem Produkt der allgemeinen Formel 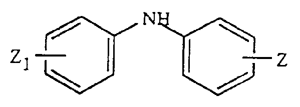 worin Z und Z₁ wie vorstehend definiert sind

## Claims

1. Process for the preparation of optionally substituted, sulphur- or selenium-containing condensed heterocycles of general formula: 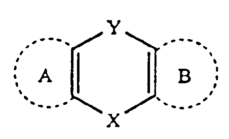 in which the rings A and B, which are identical or different, represent a phenyl ring or an aromatic heterocyclic ring containing 1 or 2 nitrogen atoms and X represents a sulphur or selenium atom and Y represents the -NH- group, characterised in that a product of general formula: 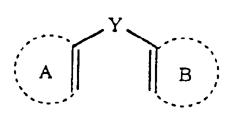 in which the rings A and B and the symbol X are defined as above, is reacted with 1/2 equivalent of sulphur or selenium per mole of phenothiazine or of phenoselenazine formed and with at least 1/2 equivalent of sulphur dioxide or of selenium dioxide per mole of phenothiazine or of phenoselenazine formed, the reaction being carried out at a temperature greater than 80°C, and the product obtained is isolated.

2. Process according to Claim 1, characterised in that the reaction is carried out in the presence of a catalyst chosen from the aluminium or gallium halides, the lithium, sodium, potassium or quaternary ammonium iodides, the iron, chromium, cobalt or copper iodides and the compounds which make possible the release of an iodide or of iodine under the reaction conditions, hydrobromic and hydriodic acids, bromine and iodine.

3. Process according to Claim 2, characterised in that the catalyst is iodine.

4. Process according to one of Claims 1, 2 or 3, characterised in that the reaction is carried out in an organic solvent chosen from saturated aliphatic or cycloaliphatic hydrocarbons containing 10 to 16 carbon atoms and nonbasic polar aprotic solvents chosen from the dialkyl sulphones containing less than 10 carbon atoms or sulpholane.

5. Process according to one of Claims 1, 2, 3 or 4, characterised in that the reaction is carried out at a temperature between 130 and 280°C.

6. Process according to one of Claims 1, 2, 3, 4 or 5, characterised in that the reaction is carried out under autogenous pressure.

7. Process according to one of Claims 1, 2, 3, 4, 5 or 6 for the preparation of phenothiazines or of phenoselenazines of general formula: 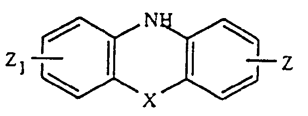 in which X represents a sulphur or selenium atom and Z and Z₁, which are identical or different, represent one or more atoms or radicals chosen from the hydrogen or halogen atoms and the alkyl, alkoxy, hydroxyl, amino, alkylamino, dialkylamino, acyl and acylamino radicals, the alkyl radicals and the alkyl portions of the other radicals containing 1 to 4 carbon atoms and possibly being substituted with a phenyl radical, by reacting sulphur and sulphur dioxide or selenium and selenium dioxide with a product of general formula: 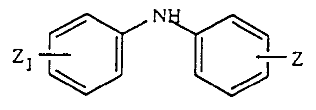 in which Z and Z₁ are defined as above.
